# FASCICULE DE BREVET EUROPEEN

(11) **EP 0 487 431 B1**
(45) Date de publication et mention de la délivrance du brevet: **02.10.1996**
(21) Numéro de dépôt: 91403172.9
(22) Date de dépôt: 25.11.1991
(51) Int. Cl.: A61K 35/78, A61K 9/16, A61K 9/50

(54) **Compositions pharmaceutiques à action laxative et leur procédé de préparation**
Pharmazeutische Laxiermittel und Verfahren
Laxative compositions and process for their preparation

(30) Priorité: 23.11.1990 FR 9014666
(43) Date de publication de la demande: 27.05.1992
(73) Titulaire: Chicouri, Marcel, F-75006 Paris (FR)
(72) Inventeur: Chicouri, Marcel, 75006 Paris (FR); Chicouri, Isabelle, 74006 Paris (FR)
(74) Mandataire: Burtin, Jean-François

(56) Documents cités:
- EP-A- 0 301 943
- FR-A- 2 616 329
- US-A- 2 111 286

## Description

La présente invention se rapporte à de nouvelles compositions pharmaceutiques à action laxative.

Elle concerne plus particulièrement une composition pharmaceutique renferment à titre de principes actifs un laxatif de lest à caractère mucilagineux et un laxatif lubrifiant.

Elle se rapporte spécifiquement à une composition pharmaceutique renferment un laxatif de lest constitué par le tégument mucilagineux pulvérulent de la graine de psyllium (Ispaghul) finement broyée et par de la paraffine liquide sous forme pulvérulente en microgranules.

On connaissait déjà par la demande de brevet français n° 87.14281 au nom du demandeur une préparation laxative du même genre où le laxatif lubrifiant était constitué d'huile de paraffine micro-encapsulée dans de la gélatine et éventuellement durcie par tannage pour obtenir une préparation pulvérulente.

Ces deux constituants tout en fournissant une préparation pharmaceutique efficace, présentaient cependant l'inconvénient de ne pas être suffisamment stables et de se détériorer au stockage. Dans ces conditions, notamment par élévation de la température dans les locaux où le médicament était conservé, de l'huile suintait rendant le médicament d'ingestion peu agréable et entraînant des éliminations d'huile intempestives par voie anale.

Ultérieurement, la demande de brevet français 87.10370 et la demande de brevet européen correspondante 0.301.943, ont décrit une préparation pharmaceutique laxative contenant une paraffine liquide micro encapsulée dans de la gélatine et de la poudre de psyllium.

Ces demandes de brevet indiquent simplement que la paraffine liquide micro-encapsulée dans la gélatine se présente sous une forme originale sèche. Il n'apparaît pas non plus que la paraffine liquide sous forme micro-encapsulée dans de la gélatine, garantisse davantage la stabilité de la préparation.

Il se trouve en outre que la densité et les caractéristiques mécaniques des deux constituants sont fondalement différentes et il en résulte une grande difficulté à obtenir un mélange homogène de poudres où les quantités de principes actifs peuvent être définies d'une manière exacte.

Il se trouve enfin que la demande de brevet français 87.10370 indique que le produit pharmaceutique laxatif est destiné à être dispersé dans un grand verre d'eau ou de jus de fruit avant l'ingestion. On voit donc à quel point la stabilité des micro capsules joue un rôle fondamental car la gélatine de ces micro capsules se dissout plus ou moins dans un véhicule aqueux et laisse passer à ce moment de la paraffine liquide.

Tous ces inconvénients ont trouvé une solution beaucoup plus satisfaisante en réalisant des micro granules de paraffine dans de la gélatine puis en durcissant la paroi de ces micro capsules et en la rendant imperméable par réaction chimique (cross linking) avec de l'adehyde glutarique. On sait, en effet, que l'adehyde glutarique est un dialdehyde apte à réagir avec des fonctions aminées en formant des bases de Schiff très peu solubles dans l'eau et peu susceptibles d'être attaquées ou digérées par des sucs digestifs en milieu acide en raison de la nature polymérique linéaire ou pontée des enchainements ainsi formées. Les combinaisons formées avec la gélatine sont ainsi beaucoup plus résistantes mécaniquement, chimiquement et physiquement. Les micro granules ainsi durcis sont difficilement altérables et leur conservation dans toutes les conditions d'hygrométrie et de température peut être raisonnablement garantie sur une longue période.

Elles ne peuvent être dégradées qu'en milieu alcalin comme par exemple dans l'intestin grêle.

En outre, pour améliorer la fluidité et la miscibilité de ces microgranules à la poudre de téguments mucilagineux, il convient de les additionner d'un excipient inerte très finement pulvérulent. Un tel excipient est de préférence une silice colloïdale comme l'Aerosil 100 ou l'Aerosil 200 ou bien une terre siliceuse purifiée comme le Syloid 244. La teneur en paraffine liquide dans de tels micro capsules est comprise entre 82 et 92% de la masse totale et de préférence entre 84 et 90%.

Pour des raisons semblables, il s'est avéré être avantageux d'incorporer à la poudre de Psyllium une alcoyl cellulose qui contribue à améliorer les propriétés physiques de la poudre de psyllium.

Parmi les alcoyl celluloses, on utilise de préférence la méthyl cellulose, l'éthyl cellulose ou l'hydroxypropyl cellulose. Le mélange alcoyl cellulose/poudre de psyllium s'effectue en pulvérisant sur la poudre de psyllium une solution de l'alcoylcellulose dans un alcanol puis en séchant le mélange ainsi formé dans un séchoir à lit fluidisé ou dans une étuve à circulation d'air forcée puis à broyer la masse ainsi obtenue et à la tamiser.

D'une manière préférée, l'alcoyl cellulose est une éthyl cellulose et en particulier une éthylcellulose ayant de 42 à 49% de groupes éthoxy, soluble dans les alcanols inférieurs. Pour l'imbibition de la poudre de Psyllium, on utilise de préférence une solution d'éthyl cellulose dans l'isopropanol à une concentration allant de 40 à 50%. La solution d'éthyl cellulose dans l'alcanol peut ensuite être diluée avec une quantité appropriée d'eau pour améliorer les qualités d'adhésivité du mélange.

La quantité d'eau incorporée à la solution alcanolique peut varier de 14 à 70% de la quantité d'alcanol mis en jeu.

Le psyllium broyé ainsi enrobé et tamisé présente une grande fluidité qui permet son incorporation aux microgranules de paraffine liquide sans risque de demixion et surtout avec l'assurance que le mélange sera parfaitement homogène et se conservera parfaitement.

Les proportions des deux principes actifs sont celles qui ont été décrites dans la demande de brevet français 87.14281 et notamment de 1 p. paraffine pour 2 p.> de tégument mucilagineux à 2 p. de paraffine pour 1 p. de tégument mucilagineux avec une préférence pour un rapport à parties égales.

Une formule préférée est celle qui renferme 3,33 g de tégument mucilagineux pulvérisé de la graine de psyllium et 3,33 g de paraffine microencapsulée, durcie par de l'aldéhyde glutarique et fluidifiée par adjonction d'un excipient inerte très finement pulvérulent.

L'exemle suivant illustre l'invention sans toutefois la limiter :

### Microcapsules de paraffine :

On introduit 3,0 kg de gélatine officinale et 87 kg d'eau. On chauffe à 65°C en agitant jusqu'à dissolution complète. On maintient la solution à cette température et on y ajoute goutte à goutte sous forte agitation la paraffine liquide de manière à obtenir des gouttelettes d'environ 10 µm. On laisse refroidir lentement la température puis plus rapidemment à 10°. On ajoute alors une solution aqueuse d'aldehyde glutarique à 50% et on maintient l'agitation pendant 30 mn. On laisse décanter les microgranules et on élimine l'excès d'aldehyde glutarique par lavage à l'eau. On sèche sommairement les microgranules puis on ajoute la silice colloidale (Aérosil 100). On mélange soigneusement puis tamise le mélange finement pulvérulent sur Tamis 200.

Les microgranules ainsi obtenus renferment 85% de paraffine liquide.

### Tégument mucilagineux de la graisse de Psyllium

Les graines de Psyllium sont criblées puis soumises à une décortication pour séparer le tégument riche en mucilage. Le tégument se détache de l'albumen. Le tégument est séparé par ventilation préférée puis est soumis à un criblage. La poudre grossière est soumise à un broyage mécanique pour obtenir une poudre demi-fine. On pulvérise sur celle-ci une solution d'éthyl cellulose dans un mélange isopropanol 25/eau 5. La masse est séchée en armoire ventilée à 40° puis broyée en grains fins. Par tamisage, on récolte la fraction qui passe entre 0.71 et 3.0 mm.

Les grains de tégument peuvent alors être mélangés avec les microcapsules de paraffine préalablement additionnées de silice colloidale. Le mélange est parfaitement homogène et ne présente au toucher ou au goût, ni grain, ni fragment important.

Cette poudre peut être répartie en sachets de 7 g environ.

## Revendications (Revendications pour l'(les) Etat(s) contractant(s) suivant(s): BE, DE, GB, IT, NL)

1. De nouvelles compositions pharmaceutiques à action laxative formées d'un laxatif de lest et d'un laxatif lubrifiant micro-encapsulé caractérisées en ce que le laxatif de lest est constitué par les téguments mucilagineux pulvérulents de la graine de Psyllium préalablement imprégnés d'une solution d'alcoylcellulose et que le laxatif lubrifiant est constitué par des microgranules de paraffine liquide, préalablement durcis par réaction chimique avec de l'aldehyde glutarique et que le mélange homogène en résultant est enrobé dans un excipient inerte très finement pulvérulent.

2. Une composition pharmaceutique selon la revendication 1 dans laquelle l'alcoylcellulose est choisie dans le groupe constitué par la méthylcellulose, l'éthylcellulose et l'hydroxypropylcellulose.

3. Une nouvelle composition selon la revendication 1 ou la revendication 2 dans laquelle l'alcoylcellulose est une éthylcellulose.

4. Une nouvelle composition selon l'une des revendications 1 à 3 dans laquelle le solvant de l'alcoylcellulose est un alcanol inférieur, éventuellement additionné d'eau.

5. Une composition selon la revendication 1 dans laquelle l'excipient inerte très finement pulvérulent est une silice colloidale.

6. Une composition selon la revendication 1 et la revendication 5 dans laquelle la teneur en paraffine liquide des microgranules durcis est comprise entre 82 et 92 % de la masse totale.

7. Une composition selon l'une des revendications 1 à 5 dans laquelle le rapport paraffine liquide sous forme micro encapsulée/tégument mucilagineux de graines de Psyllium préalablement imprégné s'échelonne de 1p pour 2p à 2p pour 1p.

8. Une composition selon la revendication 6 dans laquelle la paraffine liquide sous forme micro-encapsulée et le tégument mucilagineux des graines de Psyllium préalablement imprégné, sont à parties égales.

## Revendications (Revendications pour l'(les) Etat(s) contractant(s) suivant(s): ES)

1. Un procédé d'obtention de nouvelles compositions pharmaceutiques à action laxative formées d'un laxatif de lest et d'un laxatif micro-encapsulé caractérisé en ce que le laxatif de lest constitué par les téguments mucilagineux pulvérulents de la graine de Psyllium est au préalable imprégné d'une solution d'alcoylcellulose puis additionné au laxatif lubrifiant constitué par des microgranules de paraffine liquide préalablement durcies par réaction chimique avec l'aldéhyde glutarique et que le mélange homogène en résultant est enrobé dans un excipient inerte très finement pulvérulent.

2. Un procédé delon la revendication 1° dans lequel l'alcoylcellulose est choisie dans le groupe constitué par la méthylcellulose, l'éthylcellulose et l'hydroxypropylcellulose.

3. Un procédé selon la revendication 1° ou la revendication 2° dans lequel l'alcoylcellulose est une ethylcellulose.

4. Un procédé selon l'une des revendications 1 à 3° dans lequel le solvant de l'alcoylcellulose est un alcanol inférieur, éventuellement additionné d'eau.

5. Un procédé selon la revendication 1° dans lequel l'excipient inerte très finement pulvérulent est une silice colloidale.

6. Un procédé selon la revendication 1° et la revendication 5° dans lequel la teneur en paraffine liquide des microgranules durcies est comprise entre 82 et 92% de la masse totale.

7. Un procédé selon l'une des revendications 1 à 5° dans lequel le rapport paraffine liquide sous forme micro encapsulée/tégument mucilagineux de graines de Psyllium préalablement imprégné s'échelonne de 1p pour 2p à 2p pour 1p.

8. Un procédé selon la revendication 6° dans lequel la paraffine liquide sous forme micro-encapsulée et le tégument mucilagineux des graines de Psyllium préalablement imprégné, sont à parties égales.

## Claims (Claims for the following Contracting State(s): BE, DE, GB, IT, NL)

1. Novel pharmaceutical compositions with laxative action consisting in a bulk laxative and a micro encapsulated lubrificating laxative wherein the bulk laxative consists in the powdery mucilaginous teguments of the psyllium seeds, which have been previously be impregnated with a solution of alkylcellulose and wherein the lubrificating laxative consists in microbedlets of liquid paraffin which have been previously been hardened by chemical reaction with glutaraldelyde, and wherein the resulting the homogeneous mixture, is coated with a very finely powderous inert carrier.

2. A pharmaceutical composition according to claim 1 wherein the alkylcellulose is selected from the group consisting of methylcellulose, ethylcellulose and hydroxypropylcellulose.

3. A novel composition according to claim 1 or claim 2 wherein the alkylcellulose is an ethylcellulose.

4. A novel composition according to claims 1 to 3 wherein the solvent of the alkylcellulose is a lower alkanol, optionally added with water.

5. A composition according to claim 1 wherein the very finely powderous inert carrier is a colloidal silica.

6. A composition according to claim 1 and claim 5 wherein the content in liquid paraffin in the hardened microbedlets is comprised between 82 and 92 % of the whole mass.

7. A composition according to one of claims 1 to 5 wherein the ratio liquid paraffin in the microencapsulated form / mucilaginous teguments of Psyllium which have been previously impregnated, ranges from 1 p for 2 p to 2 p for 1 p.

8. A composition according to claim 6 wherein the liquid paraffin in the microencapsulated form and the previously impregnated mucilaginous tegument of psyllium seeds are at equal parts.

## Claims (Claims for the following Contracting State(s): ES)

1. A process for obtaining novel pharmaceutical compositions with laxative activity made of a bulk laxative and a microencapsulated laxative, characterized in that the bulk laxative consisting in the powdery mucilaginous teguments of the psyllium seed is previously impregnated with a solution of alkylcellulose then added to the lubrificating laxative made of microbedlets of liquid paraffin which have been previously hardened by chemical reaction with glutaraldehyde and in that the resulting homogeneous mixture is coated with a very finely powdery inert carrier.

2. A process according to claim 1° wherein the alkylcellulose is selected from the group consisting of methylcellulose, ethylcellulose and hydroxypropylcellulose.

3. A process according to claim 1° or claim 2° wherein the alkylcellulose is an ethylcellulose.

4. A process according to one of the claims 1 to 3° wherein the solvent of alkylcellulose is a lower alkanol, optionally added with water.

5. A process according to claim 1° wherein the very finely powdery inert excipient is a colloidal silica.

6. A process according to claim 1° and claim 5 wherein the content in liquid paraffin of the hardened microbedlets, lies between 82 and 92 % of the whole mass.

7. A process according to one of claims 1° to 5° wherein the ratio liquid paraffin in the microencapsulated form / mucilaginous teguments of the psyllium seeds which have been previously impregnated, range from 1 p for 2 p to 2 p for 1 p.

8. A process according to claim 6 wherein the liquid paraffin in the microencapsulated form and the mucilaginous teguments of the psyllium seeds which have been previous be impregnated, are at equal parts.

## Patentansprüche (Patentansprüche für folgende(n) Vertragsstaat(en): BE, DE, GB, IT, NL)

1. Neue pharmazeutische Zubereitungen mit abführende Wirkung aus einem Ballast - laxiermittel und einem mikrogekapultenen einfettenden Laxiermittel entstehenden, dadurch gekennzeichnet dass das Ballastlaxiermittel aus der pulverige, schleimige, Hülle der Samen von Psyllium, die vorher mit einer Lösung von Alkylcellulose durchgetränkt werden, bestent, and dass das einfettende Laxiermittel aus Mikrokapseln von flüssige Paraffin, die vorher durch chemische Reaktion mit Glutaraldehyd ausgehärtet werden, und dass die daraus resultierende innige Vermischung, mit einem am feinste pulverige, inerte, Träger umgehüllt wird.

2. Pharmazeutische Zubereitung nach Anspruch 1 dadurch gekennzeichnet dass die Alkylcellulose aus der Gruppe von Methylcellulose, Äthylcellulose und Hydroxypropylcellulose ausgewählt wird.

3. Neue Zubereitung nach Anspruch 1, oder Anspruch 2, worin die Alkylcellulose eine Äthylcellulose ist.

4. Neue Zubereitung nach Ansprüche 1 bis 3 worin das Lösungsmittel der Alkylcellulose, eine niedrige Alkanol dem gegebenenfalls Wasser zugemischt ist, ist.

5. Zubereitung nach Anspruch 1 worin der am feisten pulverige inerte Träger eine kolloïdales Silika ist.

6. Zubereitung nach Anspruch 1 und Anspruch 5 worin das Gehalt an flüssige Paraffin in der ausgehärtete Mikrokapseln zwischen 82 und 92 % der gesamte Masse, liegt.

7. Zubereitung nach einer der Ansprüche 1 bis 5 worin der Verhältnis flüssige Paraffin in mikrogekapselte Form, zu schleimige, Hülle der Samen von Psyllium, die vorher durchgetränkt wird, von 1T zu 2T bis 2T zu 1T verstreckt.

8. Zubereitung nach Anspruch 6 worin die flüssige Paraffin in der mikrogekapselte Form und die schleimige Hülle der Samen von Psyllium, die vorher durchgetränkte werden, auf gleiche Teile gebildet werden.

## Patentansprüche (Patentansprüche für folgende(n) Vertragsstaat(en): ES)

1. Verfahren für Herstellung neue pharmazeutische Zubereitungen mit laxierende Wirkung aus eine Ballastlaxiermittel und einem mikrogekapseltenen Laxiermittel, dadurch gekennzeichnet dass der Ballastlaxiermittel aus der pulverige schleimige Hülle der Samen von Psyllium bestehende vorher mit einer Lösung von Alkylcellulose durchgeträukt ist, denn mit dem einfittende Laxiermittel aus Mikrokapseln von flüssige Paraffin, die vorher durch chemische Reaktion mit Glutaraldehyd ansgehärtet werden, vermischt wird und dass die daraus resultierende innige Mischung mit einem am feinsten pulverige inerte Träger umgehüllt wird.

2. Verfahren nach Anspruch 1 worin die Alkylcellulose aus der Gruppe von Methylcellulose, Äthylcellulose, und Hydroxypropylcellulose ausgewählt ist.

3. Verfahren nach Anspruch 1 oder 2, worin die Alkylcellulose eine Äthylcellulose ist.

4. Verfahren nach einer der Ansprüche 1 bis 3 worin der Lösungsmittel von Alkylcellulose eine niedrige Alkanol dem gegebenenfalls Wasser zugemischt ist, ist.

5. Verfahren nach Anspruch 1, worin der am feisten pulverige Träger eine kolloïdale Silika ist.

6. Verfahren nach Anspruch 1 und Anspruch 5 worin der Gehalt an flüssige Paraffin in der ausgehärtete Mikrokapseln zwischen 82 und 92 % der gesamte Masse liegt.

7. Verfahren nach einer der Ansprüche 1 bis 5, worin der Verhältnis flüssige Paraffin in Mikrogekapselte Form, zu schleimige Hülle der Samen von Psyllium die vorher durchgetränkt werden von 1T zu 2T bis 2T zu 1T verstreckt.

8. Verfahren nach Anspruch 6 worin die flüssige Paraffin in der mikrogekapselte Form und die schleimige Hülle der Samen von Psyllium die vorher durchgetränkt werden, auf gleiche Teile gebildet werden.
